# EUROPÄISCHE PATENTANMELDUNG

(11) **EP 2 316 443 A1**
(43) Veröffentlichungstag der Anmeldung: **04.05.2011**
(21) Anmeldenummer: 10171387.3
(22) Anmeldetag: 30.07.2010
(51) Int. Cl.: A61K 31/137, A61K 47/08, A61K 47/10, A61K 47/14

(54) **Arzneimittelformulierung zur Behandlung von Nagelerkrankungen**

(30) Priorität: 30.07.2009 DE 102009035589; 25.08.2009 DE 102009038512
(71) Anmelder: Technische Universität Braunschweig, 38106 Braunschweig (DE)
(72) Erfinder: Lusiana, 38106, Braunschweig (DE); Müller-Goymann, Christel, 38104, Braunschweig (DE)
(74) Vertreter: Taruttis, Stefan Georg

(57) **Zusammenfassung**

Die Erfindung betrifft eine pharmazeutische Zusammensetzung mit einem Gehalt an Wirkstoff, der ein Antikmykotikum sein kann, so dass die Zusammensetzung zur Behandlung von Onychomykosen, insbesonderer schwerer Onychmykosen geeignet ist. Die erfindungsgemäße Formulierung ist ein bei Körpertemperatur salbenartiges und bei niedrigerer Temperatur dünnflüssiges Thermogel und ermöglicht die Permeation ausreichender Wirkstoffmengen durch menschliche Finger- und Fußnägel, um auch schwere Onychomykoscn wirksam zu behandeln.

## Beschreibung

Die Erfindung betrifft eine pharmazeutische Zusammensetzung mit einem Gehalt an pharmazeutischem Wirkstoff, die sich durch die besondere Förderung der Penetration und Permeation des Wirkstoffs in und durch Finger- und Fußnägel auszeichnet, insbesondere bei topischer Anwendung auf Nagel. Als pharmazeutischer Wirkstoff kann ein polarer, amphiphiler oder lipophiler Wirkstoff in der Zusammensetzung enthalten sein, z.B. Dithranol, vorzugsweise in Kombination mit Harnstoff, oder Vitamin-D-Präparate, z.B. Calcipotriol und/oder Tacalcitol, oder Kortikoid, z.B. Betamethason, Hydrocortison, Clobetasol, Triamcinolonacetonid, Prednicarbat und/oder Fluticason, oder Retinoide, z.B. Adapalen, Tazaroten, Acitretin, Isotretinoin und/oder Tretinoin, jeweils zur Behandlung von Nagelpsoriasis, Antiinfektiva, z.B. Clindamycin und/oder Erythromycin zur Behandlung von Nagelbettentzündung, vorzugsweise ein Antikmykotikum, so dass die zur Behandlung von Onychomykosen, insbesondere schwerer Onychmykosen geeignet ist. Die erfindungsgemäße Formulierung ermöglicht die Permeation ausreichender Wirkstoffmengen durch menschliche Finger- und Fußnägel, insbesondere von Antimykotikum, um auch schwere Onychomykosen wirksam zu behandeln und stellt daher die Formulierung bzw. pharmazeutische Zusammensetzung mit einem Gehalt an Wirkstoff, insbesondere mit Antimykotikum zur Verwendung als Medikament zur Behandlung von Nagelerkrankungen und/oder Nagelbetterkrankungen, insbesondere Onychomykosen bereit, vorzugsweise zur Applikation auf den Nagel. Entsprechend stellt die Erfindung auch die Verwendung der Formulierung mit einem Gehalt an zumindest einem der vorgenannten Wirkstoffe, insbesondere eines Antikmykotikums, zur Herstellung einer pharmazeutischen Zusammensetzung zur Verwendung für die Behandlung von Nagel- und/oder Nagelbetterkrankungen, z.B. von Onychomykosen, insbesonderer schwerer Onychmykosen bereit, besonders bevorzugt zur Behandlung mittels topischer Applikation auf einen Finger- oder Fußnagel, z.B. ausschließlich mittels topischer Applikation auf einen Finger- oder Fußnagel.

### Stand der Technik

Die EP 1390031 B1 offenbart pharmazeutische Zusammensetzungen zur topischen Anwendung, die eine Kombination aus Terbinafin mit einem zweiten Antimykotikum enthalten, und beispielsweise als Emulsion formuliert sein können. Die wässrige Phase kann als Lösungsmittel C1- bis C4-Alkohole, Polyhydroxy-C2-C5-Alkane, Poly-C2-C5-Alkylenglykole und ein wasserlösliches nicht-ionisches Tensid enthalten, jedoch kein anionisches Tensid. Als Indikationsgebiete für diese Formulierung werden durch Dermatophyten verursachte oberflächliche Mykosen genannt. Weiterhin wird zur Formulierung angegeben, dass die Wirkstoffkombination auch als Nagellack bereitgestellt werden kann. Eine Formulierung für einen entsprechenden Nagellack wird nicht angegeben.

EP 1207861 B1 beschreibt antifungizide Zusammensetzungen, in denen neben einem Antimykotikum Nährstoffe für die infektiösen Pilze enthalten sind. Als Indikation werden auch Pilzinfektionen des Nagels (Onychomykosen) genannt.

EP 0503988 B1 beschreibt eine pharmazeutische Zusammensetzung zur Verwendung gegen Onychomykosen, die Terbinafinhydrochlorid als Wirkstoff in einer wässrigen Formulierung mit einem C2-C8-Alkanol und einem penetrationsverstärkenden Mittel enthalten, das aus Glycolen, Glycolmonoethern, Glycoldiethan, Dimethylsulfoxid, Caprolactam, Dimethylisosorbid, Isopropylidenglycerin, Dimethylimidazolidinon, 2-Methylpyrrolidon-2-Pyrrolidon-2, Ethyllactat, polyoxyethylierte C8-10-Glyceride, Polyethylenglycol-20-Glycerinlaureat und Dimethylacetamid ausgewählt ist.

Die EP 0247142 B1 beschreibt filmbildende Formulierungen für Antimykotika zur Anwendung bei Onychomykosen. Die Formulierungen enthalten wässrige Mischungen aus Cellulose-Harzen, Polyethylenoxid-Harzen, Acrylsäure- und Acrylat-Harze, Vinyl-Harze und Polyvinylpyrollidon. Es wird angegeben, dass die Zusammensetzungen innerhalb von ca. 15 min auf dem Nagel trocknen.

Die WO 99/53913 beschreibt eine Vielzahl von Gelzusammensetzungen für Antimykotika zur äußerlichen Anwendung. Unter den aufgefiihrten Fungiziden ist Terbinafin nicht genannt.

Winkler und Müller-Goymann (European Journal of Pharmaceutics and Biopharmaceutics 427-437 (2005)) beschreiben unter anderem eine Formulierung, die 1 Gew.-% mittelkettige Triglyceride, 26 Gew.-% Wasser, 8% Lutrol F 127 (Poloxamer 407), 5 Gew.-% Dimethylisosorbid, 8 Gew.-% Isopropanol in Mischung mit 2,5 Gew.-% Ibuprofensäure enthält, sowie zwei ähnliche Formulierungen, in denen jedoch entweder die mittelkettigen Triglyceride oder mittelkettige Triglyceride und Ibuprofensäure fehlen, jeweils zur Permeationsverstärkung von 5-Aminolävulinsäure für die photodynamische Therapie, z.B. gegen Hautkrebs.

Die DE 10 2005 019628 beschreibt Formulierungen, die die Permeation von unter anderem Clotrimazol und Terbinafin durch die menschliche Haut verstärken, und 10-30 Gew.-% Polyetherpolyol, 2,5-10 Gew.-% Lipide, 5-20 Gew.-% Dimethylisosorbid, 5-20 Gew.-% C2-C4-Alkohol und 40-60 Gew.-% Wasser enthalten.

### Aufgabe der Erfindung

Gegenüber dem bekannten Stand der Technik ist es Aufgabe der Erfindung, eine alternative Formulierung für Wirkstoffe, insbesondere für antimykotische Wirkstoffe, bereitzustellen, die bei topischer Applikation deren Penetration durch menschliche Finger- und Fußnägel fördert, z.B. zur Behandlung von Nagel- und/oder Nagelbetterkrankungen, insbesondere von Onychomykosen.

### Allgemeine Beschreibung der Erfindung

Die Erfindung löst die Aufgabe mit den anspruchsgemäßen Merkmalen und stellt insbesondere eine Formulierung zur Verwendung als Medikament bei der Behandlung von Nagel- und/oder Nagelbetterkrankungen, z.B. von Nagelmykosen (Onychomykosen), bereit, die einen pharmazeutischen Wirkstoff in einer Formulierung auf wässriger Grundlage enthält oder aus zumindest einem Wirkstoff und der Formulierung besteht, die
- 0, 1 - 20 Gew.-% zumindest eines apolaren, lipophilen, amphiphilen, zwitterionischen oder stark polaren, pharmazeutisch annehmbaren Wirkstoffs, vorzugsweise Terbinafin, insbesondere Terbinafinhydrochlorid,
- 10 - 50 Gew.-%, vorzugsweise 10 - 30 Gew.-% eines Polyetherpolyols oder einer Mischung dieser, beispielsweise ausgewählt aus Polyethylenglycol (α-Hydro-ω-hydroxy-poly(oxy-1,2-ethandiol)), Polypropylenglycol und/oder Poloxamer (besonders bevorzugt Poloxamer 407, α-Hydro-ω-hydroxy-poly(oxyethylen)-poly(oxypropylen)-poly(polyoxyethylen)-blockcopolymer,
- 5 - 25 Gew.-% Dimethylisosorbid,
- 2,5 - 10 Gew.-% Triglyceride, vorzugsweise mittelkettige Triglyceride, insbesondere gemäß Deutschem Arzneibuch, und
- 5 - 20 Gew.-% eines C₂- bis C₄-Alkohols oder eine Mischung daraus, bevorzugt ausgewählt unter Ethanol und Isopropanol,
- Rest Wasser,
- vorzugsweise ohne Ibuprofen
aufweist oder daraus besteht, insbesondere zur Behandlung mittels topischer Applikation auf den Nagel, und wahlweise zusätzlich mit pharmazeutisch annehmbarem Farbstoff oder Parfüm versetzt ist. Die erfindungsgemäße Formulierung zur Verwendung als Medikament ist insbesondere zur Applikation auf den Nagel zur Behandlung von Nagel- und Nagelbetterkrankungen, die insbesondere Onychmykosen sind, geeignet und ist entsprechend insbesondere zur Verwendung für die Applikation auf den Nagel bei diesen Nagelerkrankungen hergerichtet. Die erfindungsgemäße Formulierung bzw. Zusammensetzung ist daher zur Verwendung bei der Behandlung von Nagel- und Nagelbetterkrankungen durch topische Applikation auf den Nagel geeignet, so dass die Formulierung z.B. zur Verwendung bei der Behandlung von Nagel- und Nagelbetterkrankungen hergerichtete ist, wobei die Behandlung die topische Applikation auf den Nagel umfasst oder daraus besteht.

Der pharmazeutische Wirkstoff ist insbesondere ein antimykotischer Wirkstoff, z.B. Terbinafin (Base) oder dessen wasserlösliches Salz Terbinafinhydrochlorid, zur Behandlung von Onychomykosen, insbesondere von schweren Onychomykosen. Zur Behandlung von Nagelpsoriasis (Schuppenflechte des Nagels) ist der pharmazeutische Wirkstoff z.B. ausgewählt aus der Gruppe, die Dithranol, vorzugsweise in Kombination mit Harnstoff, Vitamin-D-Präparate, z.B. Calcipotriol und/oder Tacalcitol (vorzugsweise in einer Konzentration <100 µg/g Formulierung), Kortikoide, z.B. Betamethason, Hydrocortison, Clobetasol, Triamcinolonacetonid, Prednicarbat und/oder Fluticason (insgesamt vorzugsweise in einer Konzentration <1 Gew.-% in der Formulierung), Retinoide, z.B. Adapalen, Tazaroten, Acitretin, Isotretinoin und/oder Tretinoin (insgesamt vorzugsweise in einer Konzentration <1 Gew.-% in der Formulierung) umfasst. Zur Behandlung von Nagelbettentzündung ist der pharmazeutische Wirkstoff z.B. ausgewählt aus der Gruppe, die Antiinfektiva, z.B. Clindamycin und/oder Erythromycin umfasst.

Bevorzugt weist die erfindungsgemäße Formulierung auf oder besteht aus 20 Gew.-% Poloxamer407 (Lutrol F127), 12,5 Gew.-% Dimethylisosorbid, 12,5 Gew.-% Isopropanol, 5 Gew.-% mittelkettige Triglyceride, 50 Gew.-% Wasser, bidestilliert, mit pharmazeutischem Wirkstoff, der vorzugsweise ein Antimykotikum ist, insbesondere zu 1 bis 10 Gew.-% in der vorgenannten Formulierung.

Überraschender Weise hat sich gezeigt, dass die Formulierung auch zur Verwendung bei der Behandlung von Onychomykosen geeignet ist, wenn der pharmazeutische Wirkstoff polar, amphiphil oder lipophil ist. Denn die Formulierung ermöglicht eine hohe Penetration bzw. Permeation auch polarer oder lipophiler pharmazeutischer Wirkstoffe durch den menschlichen Nagel, z.B. von lipophilem antimykotischem Wirkstoff, insbesondere Terbinafin (Base), und von polarem antimykotischem Wirkstoff, insbesondere Terbinafinhydrochlorid.

Entsprechend stellt die vorliegende Erfindung die Verwendung der vorgenannten Formulierung in Mischung mit zumindest einem pharmazeutischen Wirkstoff, insbesondere einem Antimykotikum, zur Verwendung bei der Herstellung eines Arzneimittels zur Verwendung bei der Behandlung von Nagelmykosen bereit, insbesondere zur topischen Applikation auf den Nagel.

Die herkömmliche Therapie von Mykosen der Haut sieht gegenwärtig die topische Anwendung von Lamisil-Dermgel (Novartis) vor, die als Wirkstoff 1% Terbinafin (Base) enthält, und die folgende Zusammensetzung aufweist:
96% Ethanol, Isopropylmyristat, Polysorbat20, Carbomer, Sorbitanlaurat, Benzylalkohol, Natriumhydroxid, Butylhydroxytoluol, gereinigtes Wasser.

Es hat sich überraschenderweise gezeigt, dass die erfindungsgemäße Formulierung eine signifikant höhere Permeation antimykotischen Wirkstoffs, insbesondere von Terbinafin, das z.B. als Hydrochlorid enthalten sein kann, durch den Nagel bewirkt, und daher zur Verwendung für die Behandlung von Nagel- und/oder Nagelbetterkrankungen, die insbesondere Onychomykosen sind, durch topische Applikation auf den Nagel besonders geeignet ist.

Insbesondere ist die erfindungsgemäße Formulierung zur Behandlung schwerer Nagelmykosen geeignet, da die Permeation durch den Nagel so hoch ist, dass auch schwere Nagelmykosen behandelbar sind. Dies steht im Unterschied zu den bisherigen Therapien, bei denen die topische Anwendung, beispielsweise von Lamisil, auf das Auftragen auf die Haut beschränkt ist, während das Antimykotikum für schwächere Nagelmykosen in einer Formulierung als Nagellack eingesetzt wird, bei schweren Nagelmykosen jedoch eine systemische Verabreichung des Antimykotikums, insbesondere von Terbinafin erfolgt. Denn die bisher für die Therapie von Nagelmykosen eingesetzten Formulierungen von Terbinafin ergeben keine ausreichend hohe Permeation durch den Nagel, um schwere Nagelmykosen wirksam zu behandeln.

Neben der penetrationsverstärkenden Wirkung durch Nagel hat die erfindungsgemäße Formulierung den Vorteil, bei Raumtemperatur eine wesentlich höhere Konsistenz, z.B. fest oder halbfest aufzuweisen, als bei tiefen Temperaturen, wo sie beispielsweise bei 5-12 °C flüssig ist. Diese Gelierung bei höheren Temperaturen, für die Zwecke der Erfindung auch als thermoreversible Gelierung bezeichnet, ist in Kombination mit der penetrationsverstärkenden Wirkung für pharmazeutische Wirkstoffe, z.B. für antimykotische Wirkstoffe, durch den Nagel besonders vorteilhaft, da ein einfaches Einmischen von Wirkstoff in die Formulierung bei niedrigen Temperaturen möglich ist, während bei höheren Temperaturen, insbesondere bei Körpertemperatur die höhere Konsistenz der Formulierung den Verbleib der Formulierung am Applikationsort, d.h. auf dem Nagel begünstigt, und damit die kontinuierliche Permeation von Wirkstoff durch den Nagel verstärkt, insbesondere gegenüber herkömmlichen Salbenformulierungen, die bei Körpertemperatur dünnflüssig werden und vom Applikationsort verlaufen, bzw. von einem Verband aufgesogen werden und nicht mehr für die Permeation zur Verfügung stehen. Vorzugsweise ist die erfindungsgemäße Formulierung daher bei niedrigen Temperaturen, z.B. bei 5 bis 10 °C signifikant weniger viskos, z.B. dünnflüssig, als bei um 10 bis 20 °C höheren Temperaturen, z.B. bei 15 bis 30 °C, bei denen die Formulierung signifikant viskoser ist, z.B. cremeartig, halbfest oder fest.

Die erfindungsgemäße Formulierung enthält vorzugsweise keinen Haftungsverbesserer, keinen Filmbildner, kein Tensid und auch kein Keratolytikum.

Die erfindungsgemäße Formulierung weist ein Polyetherpolyol im Bereich von 10 - 50 Gew.-%, vorzugsweise 15 - 25 Gew.-%, besonders bevorzugt 20 Gew.-% auf. Das Polyetherpolyol kann unter Polyoxyethylenglycolen mit einem Molekulargewicht im Bereich von 1000 bis 30.000, Polyoxypropylenglycolen im Bereich von 1000 bis 30.000, Polyoxyethylenpolyoxypropylen-Blockcopolymeren mit einem Molekulargewicht im Bereich von 1000 bis 30.000 und Mischungen dieser ausgewählt werden. Als Polyoxyethylenglycol können auch Polyethylenglycole verwendet werden, bevorzugt beispielsweise Polyethylenglycol 600 (PEG - 12), Polyethylenglycol 400 (PEG - 8) und Polyethylenglycol 300 (PEG - 6).

Besonders bevorzugte Polyetherpolyole sind Poloxamere mit einem Molekulargewicht im Bereich von ca. 2000 bis 20.000 mit einem Anteil an Oxyethylengruppen im Bereich von 40 bis 90 Gew.-%, beispielsweise Poloxamer 124, Poloxamer 188, Poloxamer 237 und Poloxamer 338, wie in Ph. Eur. definiert. Ein besonders bevorzugtes Polyetherpolyol ist Poloxamer 407.

Weiterhin enthält die erfindungsgemäße Formulierung Dimethylisosorbid im Bereich von 5 - 25 Gew.-%, vorzugsweise im Bereich von 10 - 20 Gew.-% oder bis 15 Gew.-%, besonders bevorzugt 12 - 13 Gew.-%.

Ein weiterer Bestandteil der erfindungsgemäßen Formulierung sind Lipide, ausgewählt unter mittelkettigen Triglyceriden, fetten Ölen, Paraffinöl, flüssigen Wachsen, Isopropylmyristat, Cetyloleat und Mischungen dieser im Bereich von 2,5 - 10 Gew.-%., bevorzugter 4 - 6 Gew.-%. Besonders bevorzugt sind mittelkettige Triglyceride, beispielsweise entsprechend der Definition des Europäischen Arzneibuchs (Ph. Eur., 4. Ausgabe, Grundwerk 2002), d.h. ein Gemisch von Triglyceriden gesättigter Fettsäuren, hauptsächlich Caprylsäure und Caprinsäure, die zu mindestens 95 % gesättigte Fettsäuren mit 8 bis 10 Kohlenstoffatomen aufweisen. Besonders bevorzugt ist eine Fettsäurefraktion folgender Zusammensetzung: Capronsäure zu maximal 2 %, Caprylsäure zu 50 bis 80 %, Caprinsäure zu 20 bis 50 %, Laurinsäure zu höchstens 3% und Myristinsäure zu höchstens 1 %.

Weiter enthält die erfindungsgemäße Formulierung ca. 5 - 20 Gew.-%, bevorzugt ca. 10 - 15 Gew.-%, besonders bevorzugt ca. 12 - 13 Gew.-% eines C₂- bis C₄-Alkohols, vorzugsweise Ethanol, Propanol, Butanol, tert. Butanol, besonders bevorzugt Isopropanol.

Die erfindungsgemäße Zusammensetzung enthält weiterhin ca. 40 - 60 Gew.-% Wasser, bevorzugt ca. 45 - 55 Gew.-% Wasser, besonders bevorzugt ca. 50 Gew.-% Wasser. Die erfindungsgemäße Formulierung kann als Wirkstoff ein oder mehrere Antimykotika enthalten, insbesondere amphiphile, zwitterionische und stark polare Antimykotika. Ein erfindungsgemäß bevorzugter Wirkstoff ist Terbinafin, insbesondere dessen Hydrochlorid, in einem Anteil von ca. 0, 1 - 20 Gew.-%, besonders bevorzugt ca. 5 - 15 Gew.-%, am bevorzugtesten ca. 10 Gew.-% an der Gesamtzusammensetzung.

Das Herstellungsverfahren beruht auf der Mischung der Formulierungsbestandteile, wobei vorteilhafterweise keine Vorbehandlungschritte einzelner Bestandteile, beispielsweise Erwärmung zur Verflüssigung, erforderlich sind.

So kann das Polyetherpolyol als Feststoff vorgelegt werden, zu dem dann Dimethylisosorbid (flüssig) zugegeben wird und anschließend das Lipid, beispielsweise mittelkettige Triglyceride (flüssig). Anschließend wird der C₂- bis C₄-Alkohol (flüssig) zugegeben, schließlich Wasser. Durch einfaches mechanisches Mischen bei Raumtemperatur, beispielsweise Rühren mit einem mechanischen hochtourigen Rührer, z.B. einem Unguator^{®}, kann die erfindungsgemäße Formulierung hergestellt werden, wobei die Mischung unmittelbar während des Rührvorgangs geliert.

Die gemischte, gelierte Zubereitung ist z.B. über Nacht oder länger bei Raumtemperatur lagerstabil. Pharmazeutische Wirkstoffe können durch Rühren eingearbeitet werden. Geeignete Antimykotika sind Azolantimykotika, insbesondere Bifonazol, Clotrimazol, und Econazol, Squalenoxidasehemmer-Antimykotika, insbesondere Naftitin, Morpholinderivat-Antimykotika, insbesondere Amorolfin, sowie Terbinafin, Nystatin, Griseofulvin, Flucytosin, Ciclopirox und Mischungen dieser.

Das erfindungsgemäße Herstellungsverfahren kommt vorteilhafterweise mit einem einfachen hochtourigen Rührwerk zum Herstellen der Mischung aus, beispielsweise einem Apotheken-üblichen Unguator, so dass die Formulierung auch ohne zusätzlichen Bedarf an Geräten in Apotheken-üblichen Mengen hergestellt werden kann. Das einfache Herstellungsverfahren der Erfindung bietet auch in der großtechnischen Produktion Vorteile, da keine Bestandteile besonders vorbehandelt werden müssen, beispielsweise Verflüssigungen durch Erwärmen entbehrlich sind.

Als Alternative kann das Herstellungsverfahren die Schritte des Zusammenfügens der Formulierungsbestandteile Polyetherpolyol, Dimethylisosorbid, C₂ - C₄-Alkohol und Wasser, also noch ohne Lipid oder Wirkstoff, in einem Behälter aufweisen und beispielsweise in einer Fantaschale durchgeführt werden. Bei Lagerung bei niedriger Temperatur, z.B. 4 °C über Nacht ergibt sich eine klare viskose Lösung. Dieser Lösung kann, vorzugsweise nach Erwärmen auf Raumtemperatur der Lipidanteil zugesetzt und durch Rühren eingearbeitet werden. Die Mischung wird trübe und bei weiterer Erwärmung, z.B. durch das Rühren selbst, verfestigt sich die Mischung zu einem thermoreversiblen Gel. In dieses Gel kann dann der Wirkstoff durch Rühren eingebracht werden.

Die erfindungsgemäße Formulierung ist lagerfähig und kann daher vorteilhafterweise unabhängig von dem später einzubringenden Wirkstoff hergestellt und ohne Kühlung, z.B. oberhalb von 13 °C gelagert werden. Aufgrund der thermoreversiblen Gelierung der erfindungsgemäßen Formulierung ist die Einbringung von Wirkstoffen einfach handhabbar, da die Wirkstoffe durch einfaches mechanisches Rühren in die Formulierung zu integrieren sind. Falls gewünscht kann die Viskosität der erfindungsgemäßen Formulierung durch Kühlen, beispielsweise auf Temperaturen von 5 - 12 °C erniedrigt werden. Durch Erwärmen der nach Einmischen eines Wirkstoffs wirkstoffhaltigen Formulierung auf Raumtemperatur wird die gewünschte Viskosität von cremeartig oder halbfest bis fest erreicht, ohne dass weitere Bearbeitungschritte erforderlich wären. Diese Viskositätserhöhung bei höheren Temperaturen ist abhängig von Art und Anteil des eingemischten Wirkstoffs, wobei die temperaturabhängige Viskositätserhöhung der Formulierung für einen eingemischten Wirkstoff stoff- und mengenspezifisch bestimmbar ist.

Die Formulierung ist sowohl zur Verwendung zur Behandlung von Nagel- und Nagelbetterkrankungen des Menschen als auch zur Behandlung von Nagel- und Nagelbetterkrankungen von Tieren, insbesondere von Paarhufern und Unpaarhufern verwendbar, insbesondere beim Rind, beim Schaf, bei der Ziege, beim Pferd, beim Schwein, beim Kamel, beim Elefant, beim Nilpferd, beim Hund oder bei der Katze. Eine bevorzugt zu behandelnde Nagelerkrankung ist eine Nagelmykose.

### Genaue Beschreibung der Erfindung

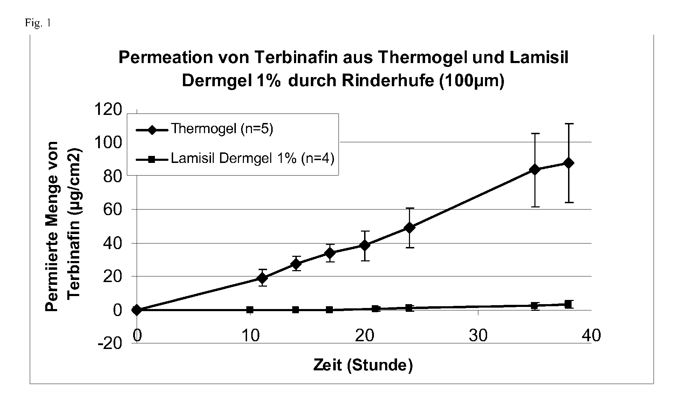

Die Erfindung wird nun anhand von Beispielen genauer mit Bezug auf die Figur beschrieben, die
- in Figur 1 die graphische Auswertung der durch eine Rinderhufscheibe permeierten Menge Terbinafinhydrochlorid für eine erfindungsgemäße Formulierung (◆) und für Lamisil Dermgel 1% (■) zeigt.

### Beispiel 1: Herstellung der erfindungsgemäßen Formulierung mit Antimykotikum

In eine herkömmliche, Apotheken-übliche Unguatorkruke als Mischgefäß werden bei Raumtemperatur die folgenden Bestandteile eingewogen:
20,0 g Poloxamer 407 (Lutrol F 127, Bayer), 12,5 g Dimethylisosorbid (flüssig), 5,0 g mittelkettige Triglyceride (flüssig, entsprechend Europäischem Arzneibuch, Ph. Eur.), 12,5 g Isopropanol und schließlich 50,0 g Wasser mit Terbinafinhydrochlorid zu 1 Gew.-% an der Gesamtmischung.

Durch die Öffnung der Kruke wird ein mechanischer Rührer eingeführt und die Zusammenstellung durch Rühren mit einem Unguator^{®} bei 5°C gemischt. Die zunächst flüssige Mischung geliert unmittelbar während des Rührens bei ca. 1450 Upm für 1,5 min. Die fertig gemischte, gelartige Zubereitung wird über Nacht bei Raumtemperatur gelagert. Der pH der Lösung bzw. der gelartigen Mischung lässt sich auf jeden gewünschten Wert einstellen.

### Beispiel 2: Messung der Permeation von Wirkstoff aus der erfindungsgemäßen Formulierung durch Nagel

Als Beispiel für einen menschlichen Finger- oder Fußnagel wurden Rinderhufscheiben von 100µm Dicke verwendet. Der Flux durch das Nagelmodell wurde folgendermaßen gemessen:
Rinderhufscheiben wurden durch Ausschneiden rechteckiger Stücke von ca. 2x2cm aus den Sohlen der Hufe, Äquilibrieren der ausgeschnittenen Stücke über Nacht in Wasser, Schneiden in Scheiben von ca. 100µm Dicke mit dem Mikrotom und Ausstanzen von Abschnitten von ca. 15mm Durchmesser aus den Scheiben und anschließenden Äquilibrieren in PBS für 10 h vorbereitet. In modifizierten Franz-Diffusionszellen wurde die Diffusion bei 32°C gemessen: Als Akzeptor wurde phosphatgepufferte Salzlösung (PBS), pH 7,4, in einem abgemessenen Volumen von 5,2 - 6,3 mL vorgelegt. Zwischen Akzeptorkammer und die Donorkammer wurden die äquilibrierten Rinderhufscheiben (Permeationsflächen von 0,48 -0,6cm²) befestigt. Erfindungsgemäßes Thermogel bzw. Lamisil wurden in Donorkammern eingebracht. Der Probenzug betrug je 100µL, und der Akzeptor wurde anschließend mit frischem PBS ergänzt. Nach einer Permeation für 40h bei Raumtemperatur wurde die Konzentration des Terbinafins in der Akzeptorkammer mittels HPLC (Säule Zorbax SB-C18, Agilent; Fließmittel: Methanol:Wasser 95:5 mit 10mM Triethylamin bei Raumtemperatur, isokratisch; Flußrate 1,5mL/min, Detektion bei 254nm, 40µL injiziertes Probenvolumen) bestimmt.

Das Ergebnis ist in Figur 1 gezeigt, aus der deutlich wird, dass die erfindungsgemäße Formulierung eine deutlich höhere Permeation durch Nagel ergibt, als die Vergleichszusammensetzung Dermgel.

Aus den Messwerten konnten die folgenden Daten für das Antimykotikum ermittelt werden:

| | Antimykotikum | | | |
|---|---|---|---|---|
| Formulierung | Flux (g/(cm² s)) | akkumulierte Menge / Fläche in Hufen nach 40h (µg/cm²) | Gesamtmenge Terbinafin permeiert und penetriert (µg) | Verzögerungszeit (min) |
| erfindungsgemäßes Thermogel (n=5) | 6,59 x 10⁻¹⁰ +/- 1,59 x 10⁻¹⁰ | 20,49 +/- 1,48 | 65,44 +/- 8,73 | 222,26 +/- 45,24 |
| Lamisil Dermgel 1% | 3,54 x10⁻¹¹ +/- 1,67 x10⁻¹¹ | 5,65 +/- 0,93 | 5,78 +/- 0,94 | 485,54 +/- 291,53 |

Diese Daten zeigen am Beispiel eines antimykotischen Wirkstoffs, dass der Flux an pharmazeutischem Wirkstoff durch Nagel mit der erfindungsgemäßen Formulierung wesentlich höher ist, und die Verzögerungszeit (*lag*) deutlich kürzer ist, so dass mit der erfindungsgemäßen Formulierung schneller mehr pharmazeutischer Wirkstoff durch Nagel transportiert wird, und bei topischer Applikation auf den Nagel eine effektive Behandlung von Nagelerkrankungen und Nagelbetterkrankungen erbringt, insbesondere von Nagelmykosen einschließlich schwerer Nagelmykosen.

Auch die in dem Nagelmodell akkumulierte Menge des als Beispiel für einen Wirkstoff verwendeten Antimykotikums und die Gesamtmenge des Antimykotikums, das in das und durch das Nagelmodell penetriert bzw. permeiert war, zeigen die wesentlich höheren effektiven Konzentrationen des beispielhaften Wirkstoffs Antimykotikum, die mittels der erfindungsgemäßen Formulierung eingebracht werden.

## Patentansprüche

1. Formulierung zur Verwendung als Medikament zur Applikation auf den Nagel zur Behandlung von Nagelerkrankungen und/oder Nagelbetterkrankungen, wobei die Formulierung
- 0, 1 - 20 Gew.-% zumindest eines apolaren, lipophilen, amphiphilen, zwitterionischen oder stark polaren, pharmazeutisch annehmbaren Wirkstoffs,
- 10 - 50 Gew.-% eines Polyetherpolyols oder einer Mischung dieser, beispielsweise ausgewählt aus Polyethylenglycol (α-Hydro-ω-hydroxy-poly(oxy-1,2-ethandiol)), Polypropylenglycol und/oder Poloxamer (besonders bevorzugt Poloxamer 407, α-Hydro-ω-hydroxy-poly(oxyethylen)-poly(oxypropylen)-poly(polyoxyethylen)-blockcopolymer,
- 5 - 25 Gew.-% Dimethylisosorbid,
- 2,5 - 10 Gew.-% Triglyceride, vorzugsweise mittelkettige Triglyceride, insbesondere gemäß Deutschem Arzneibuch, und
- 5 - 20 Gew.-% eines C₂- bis C₄-Alkohols oder eine Mischung daraus, bevorzugt ausgewählt unter Ethanol und Isopropanol,
- Rest Wasser aufweist.

2. Formulierung nach Anspruch 1, **dadurch gekennzeichnet, dass** der Wirkstoff aus der Gruppe ausgewählt ist, die antimykotische Wirkstoffe umfasst, und die Nagel-und/oder Nagelbetterkrankung eine Onychomykose ist.

3. Formulierung nach Anspruch 2, **dadurch gekennzeichnet, dass** der antimykotische Wirkstoff ein Azolantimykotikum, ein Squalenoxidasehemmer-Antimykotikum, ein Morpholinderivat-Antimykotikum, Terbinafin, Nystatin, Griseofulvin, Flucytosin, und/oder Ciclopirox ist.

4. Formulierung nach Anspruch 1, **dadurch gekennzeichnet, dass** der Wirkstoff aus der Gruppe ausgewählt ist, die gegen Nagelpsoriasis wirksame Wirkstoffe umfasst, und die Nagel- und/oder Nagelbetterkrankung Nagelpsoriasis ist.

5. Formulierung nach Anspruch 1, **dadurch gekennzeichnet, dass** der Wirkstoff aus der Gruppe ausgewählt ist, die gegen Nagelbettentzündung wirksame Wirkstoffe umfasst, und die Nagel- und/oder Nagelbetterkrankung eine Nagelbettentzündung ist.

6. Formulierung nach einem der voranstehenden Ansprüche, **dadurch gekennzeichnet, dass** die Formulierung aus
- 0, 1 - 20 Gew.-% zumindest eines apolaren, lipophilen, amphiphilen, zwitterionischen oder stark polaren, pharmazeutisch annehmbaren Wirkstoffs,
- 10 - 50 Gew.-% eines Polyetherpolyols oder einer Mischung dieser, beispielsweise ausgewählt aus Polyethylenglycol (α-Hydro-ω-hydroxy-poly(oxy-1,2-ethandiol)), Polypropylenglycol und/oder Poloxamer (besonders bevorzugt Poloxamer 407, α-Hydro-ω-hydroxy-poly(oxyethylen)-poly(oxypropylen)-poly(polyoxyethylen)-blockcopolymer,
- 5 - 25 Gew.-% Dimethylisosorbid,
- 2,5 - 10 Gew.-% Triglyceride, vorzugsweise mittelkettige Triglyceride, insbesondere gemäß Deutschem Arzneibuch, und
- 5 - 20 Gew.-% eines C₂- bis C₄-Alkohols oder eine Mischung daraus, bevorzugt ausgewählt unter Ethanol und Isopropanol,
- Rest Wasser, Farb- und/oder Parfümstoff besteht.

7. Formulierung nach einem der voranstehenden Ansprüche, **dadurch gekennzeichnet, dass** die Formulierung zur topischen Applikation auf den Nagel hergerichtet ist.

8. Verwendung einer Formulierung, die
- 0, 1 - 20 Gew.-% zumindest eines apolaren, lipophilen, amphiphilen, zwitterionischen oder stark polaren pharmazeutisch annehmbaren Wirkstoffs,
- 10 - 50 Gew.-% eines Polyetherpolyols oder einer Mischung dieser, beispielsweise ausgewählt aus Polyethylenglycol (α-Hydro-ω-hydroxy-poly(oxy-1,2-ethandiol)), Polypropylenglycol und/oder Poloxamer (besonders bevorzugt Poloxamer 407, α-Hydro-ω-hydroxy-poly(oxyethylen)-poly(oxypropylen)-poly(polyoxyethylen)-blockcopolymer,
- 5 - 25 Gew.-% Dimethylisosorbid,
- 2,5 - 10 Gew.-% Triglyceride, vorzugsweise mittelkettige Triglyceride, insbesondere gemäß Deutschem Arzneibuch, und
- 5 - 20 Gew.-% eines C₂- bis C₄-Alkohols oder eine Mischung daraus, bevorzugt ausgewählt unter Ethanol und Isopropanol,
- Rest Wasser
aufweist oder daraus besteht, zur Herstellung einer pharmazeutischen Zusammensetzung zur Verwendung bei der Behandlung einer Nagel- und/oder Nagelbetterkrankung mittels Applikation auf den Nagel.

9. Verwendung einer Formulierung nach Anspruch 8, **dadurch gekennzeichnet, dass** der Wirkstoff aus der Gruppe ausgewählt ist, die antimykotische Wirkstoffe umfasst, und die Nagel- und/oder Nagelbetterkrankung eine Onychomykose ist.

10. Verwendung einer Formulierung nach Anspruch 9, **dadurch gekennzeichnet, dass** der antimykotische Wirkstoff ein Azolantimykotikum, ein Squalenoxidasehemmer-Antimykotikum, ein Morpholinderivat-Antimykotikum, Terbinafin, Nystatin, Griseofulvin, Flucytosin, und/oder Ciclopirox ist.

11. Verwendung einer Formulierung nach Anspruch 8, **dadurch gekennzeichnet, dass** der Wirkstoff aus der Gruppe ausgewählt ist, die gegen Nagelpsoriasis wirksame Wirkstoffe umfasst, und die Nagel- und/oder Nagelbetterkrankung Nagelpsoriasis ist.

12. Verwendung einer Formulierung nach Anspruch 8, **dadurch gekennzeichnet, dass** der Wirkstoff aus der Gruppe ausgewählt ist, die gegen Nagelbettentzündung wirksame Wirkstoffe umfasst, und die Nagel- und/oder Nagelbetterkrankung eine Nagelbettentzündung ist.

13. Verwendung einer Formulierung nach einem der Ansprüche 8 bis 12, **dadurch gekennzeichnet, dass** die Nagel- und/oder Nagelbetterkrankung beim Menschen oder beim Tier auftritt.

14. Verwendung einer Formulierung nach einem der Ansprüche 8 bis 13, **dadurch gekennzeichnet, dass** die Formulierung kein Ibuprofen aufweist.
